# EUROPEAN PATENT SPECIFICATION

(11) **EP 4 259 281 B1**
(45) Date of publication and mention of the grant of the patent: **04.06.2025**
(21) Application number: 21844075.8
(22) Date of filing: 10.12.2021
(51) Int. Cl.: A61P 31/12, A61K 36/48, A61K 36/53

(54) **OHWIA CAUDATA EXTRACTS FOR USE IN TREATING A CORONAVIRUS INFECTION**
OHWIA CAUDATA-EXTRAKTE ZUR VERWENDUNG BEI DER BEHANDLUNG EINER CORONAVIRUS-INFEKTION
EXTRAITS D'OHWIA CAUDATA POUR UTILISATION DANS LE TRAITEMENT D'UNE INFECTION À CORONAVIRUS

(30) Priority: 14.12.2020 US 202063124939 P
(43) Date of publication of application: 18.10.2023
(73) Proprietor: Xie Li Products Co., Ltd., Da'an Dist Taipei City, 106 (TW)
(72) Inventor: SHIH, Cheng-Yen, Taipei City, 106 (TW); LIN, Pi-Yu, Taipei City, 106 (TW); LIN, Shinn-Zong, Taipei City, 106 (TW); HUANG, Chih-Yang, Taipei City, 106 (TW); HO, Tsung-Jung, Taipei City, 106 (TW); CHIANG, Chien-Yi, Taipei City, 106 (TW); LIN, Yu-Jung, Taipei City, 106 (TW); SHIBU, Marthandam Asokan, Taipei City, 106 (TW); LIM, Wai-Ling, Selangor 47301 (MY)
(74) Representative: Potter Clarkson
(86) International application number: PCT/MY2021/050118
(87) International publication number: WO 2022/131903

(56) References cited:
- KWON EUN BIN ET AL: "Protective Effect of Flavonoids from Ohwia caudata against Influenza a Virus Infection", MOLECULES, vol. 25, no. 19, 24 September 2020 (2020-09-24), pages 4387, XP055886830, DOI: 10.3390/molecules25194387
- SHAHIDUL ALAM M. ET AL: "HIV-inhibitory diterpenoid from Anisomeles indica", FITOTERAPIA, vol. 71, no. 5, 1 September 2000 (2000-09-01), IT, pages 574 - 576, XP055886840, ISSN: 0367-326X, Retrieved from the Internet <URL:https://www.sciencedirect.com/science/article/pii/S0367326X00001970/pdfft?md5=e0ed687bfa9ba7992e28835b50471642&pid=1-s2.0-S0367326X00001970-main.pdf> DOI: 10.1016/S0367-326X(00)00197-0
- MONDAL PRIYA ET AL: "Traditional medicinal plants against replication, maturation and transmission targets of SARS-CoV-2: computational investigation", JOURNAL OF BIOMOLECULAR STRUCTURE & DYNAMICS, 5 November 2020 (2020-11-05), US, pages 1 - 18, XP055886836, ISSN: 0739-1102, Retrieved from the Internet <URL:https://www.ncbi.nlm.nih.gov/pmc/articles/PMC7651333/pdf/TBSD_0_1842246.pdf> DOI: 10.1080/07391102.2020.1842246
- MA KE-JIA ET AL: "Analgesic, anti-inflammatory, and antipyretic activities of the ethanol extract from Desmodium caudatum", PHARMACEUTICAL BIOLOGY, vol. 49, no. 4, 24 March 2011 (2011-03-24), NL, pages 403 - 407, XP055888865, ISSN: 1388-0209, DOI: 10.3109/13880209.2010.520322

## Description

### TECHNICAL FIELD

The present disclosure relates to herbal compositions, and in particular relates to herbal compositions for preventing or treating viral infections caused by a coronavirus.

### BACKGROUND

Viral infection has been established and remains as a serious animal and human affliction. Coronaviruses (CoVs) are a large family of viruses that can cause illness ranging from the common cold to more severe diseases. For example, infections with the human coronavirus strains, CoV-229E, CoV-OC43, CoV-NL63, and CoV-HKU1, usually result in mild, self-limiting upper respiratory tract infections, such as a common cold, e.g., runny nose, sneezing, headache, cough, sore throat, and fever. Other infections may result in more severe diseases, such as severe acute respiratory syndrome (SARS), Middle East respiratory syndrome (MERS), and severe acute respiratory syndrome coronavirus 2 (SARS-CoV-2), which cause severe acute respiratory syndrome, kidney failure, and death, and have triggered a global public health emergency. Particularly, coronavirus disease 2019 (COVID-19) caused by SARS-CoV-2 has infected more than 194 million people and caused over 4,160,000 deaths worldwide.

The fastest remedy to save lives thus far is the repurposing of existing FDA-approved drugs originally targeting other diseases for COVID-19. However, most antiviral drugs cause serious side effects such as nausea, diarrhea, dizziness, and fever, and there is still no specific antiviral treatment available or proven to be effective to treat or prevent coronavirus infection in a subject.

Therefore, there exists an unmet need to provide an effective and safe therapeutics to prevent or treat coronavirus infections.

### SUMMARY

In view of the foregoing, the present disclosure provides an herbal composition that is capable of interfering with the interaction between coronavirus spike (S) protein and ACE2 on the host cell surface, and suppressing the expression of proteins that are necessary for entry and replication of coronavirus in the host, thereby protecting a subject from a viral infection.

In at least one embodiment of the present disclosure, the herbal composition comprises an extract from an herbal raw material and a pharmaceutically acceptable carrier thereof, wherein the herbal raw material comprises *Ohwia caudata.* The *Ohwia caudata* is an *Ohwia caudata* root, an *Ohwia caudata* leaf, or a combination thereof.

In at least one embodiment of the present disclosure, the herbal composition comprises an extract from an herbal raw material comprising *Ohwia caudata,* and at least one of *Artemisia argyi, Ophiopogon japonicus, Houttuynia cordata, Platycodon grandiflorus, Glycyrrhiza uralensis, Perilla frutescens,* and chrysanthemum. In some embodiments, the herbal composition comprises an extract from an herbal raw material comprising *Ohwia caudata,* and *Artemisia argyi, Ophiopogon japonicus, Houttuynia cordata, Platycodon grandiflorus, Glycyrrhiza uralensis, Perilla frutescens,* and chrysanthemum. The extract from the herbal raw material is a water extract.

In at least one embodiment of the present disclosure, the herbal composition comprises an extract from an herbal raw material comprising, based on a total weight thereof, 18% to 25% by weight of *Ohwia caudata,* and at least one of 18% to 25% by weight of *Artemisia argyi,* 10% to 17% by weight of *Ophiopogon japonicus,* 10% to 17% by weight of *Houttuynia cordata,* 10% to 17% by weight of *Platycodon grandiflorus,* 4% to 11% by weight of *Glycyrrhiza uralensis,* 4% to 11% by weight of *Perilla frutescens,* and 0.4% to 11% by weight of chrysanthemum.

The herbal composition may be prepared by a method comprising: providing the herbal raw material as mentioned above; extracting the herbal raw material with an extracting solution to obtain a crude extract, wherein the extracting solution comprises water; and removing solid from the crude extract to obtain a liquid portion.

The method for preparing the herbal composition may further comprise crushing the herbal raw material into powder or pieces.

Extracting the herbal raw material may comprise boiling the herbal raw material in the extracting solution for at least 5 minutes, such as 5 minutes to 2 hours, and/or immersing the herbal raw material in the extracting solution with a temperature lower than a boiling point thereof for at least 10 minutes, such as 10 minutes to 1 hour. The weight ratio of the herbal raw material to the extracting solution may be from 2:1 to 30:1, such as 2:1, 3:1, 5:1, 8:1, 10:1, 15:1, 20:1, 25:1 and 30:1.

The method for preparing the herbal composition may further comprise concentrating the liquid portion to obtain a concentrated extract.

The herbal composition of the invention is for use in for preventing or treating a viral infection caused by a coronavirus.

In some embodiments, the coronavirus is SARS-CoV, MERS-CoV, SARS-CoV-2, mouse hepatitis virus (MHV), or porcine epidemic diarrhea virus (PEDV). In some embodiments, the coronavirus is a variant of SARS-CoV-2, such as a D614G mutant strain, a B.1.1.7 (Alpha) mutant strain, a B.1.351 (Beta) mutant strain, and a P1 mutant strain.

In at least one embodiment of the present disclosure, the extract from the herbal raw material in the herbal composition is administered to the subject in an effective amount of from about 25 mg/kg/day to about 2,500 mg/kg/day, such as from about 30 mg/kg/day to about 1,000 mg/kg/day, and from about 50 mg/kg/day to about 500 mg/kg/day.

In the present disclosure, the herbal composition provided in the present disclosure as an antivirus agent may inhibit virus replication and reduce the amount of viruses in a host cell. In addition, the herbal composition provided in the present disclosure is safe and may solve the prior-art problems of side effects. Hence, the present disclosure provides an effective strategy against viral infections, which is useful in controlling the outbreak of coronaviruses. The invention is defined by the appended claims.

### BRIEF DESCRIPTION OF THE DRAWINGS

The present disclosure can be more fully understood by reading the following descriptions of the embodiments, with reference made to the accompanying drawings.

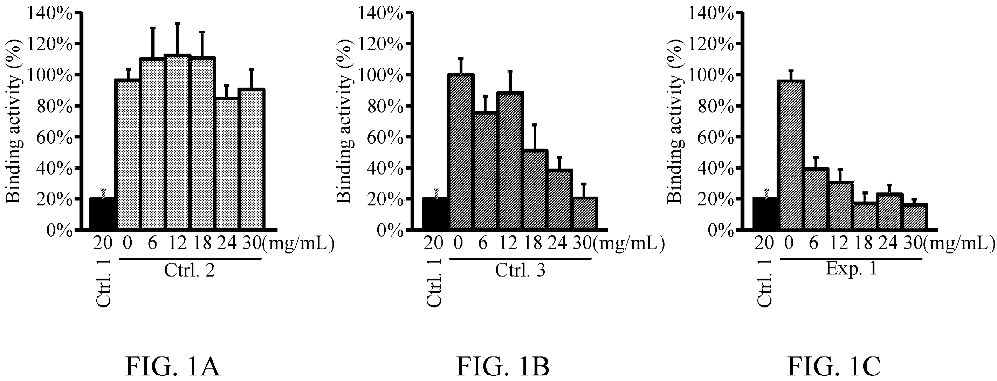

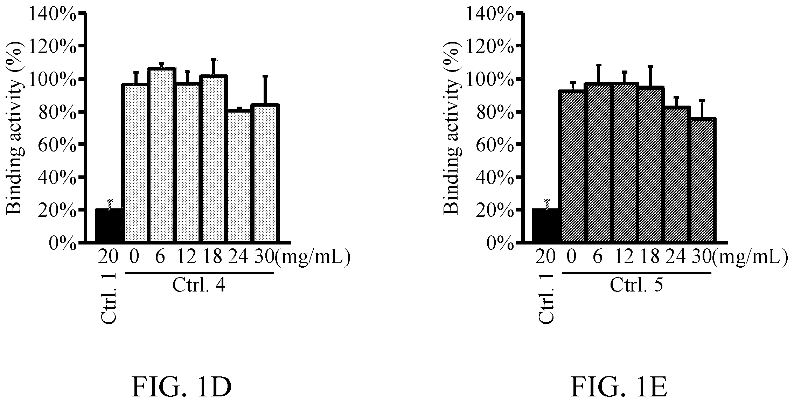

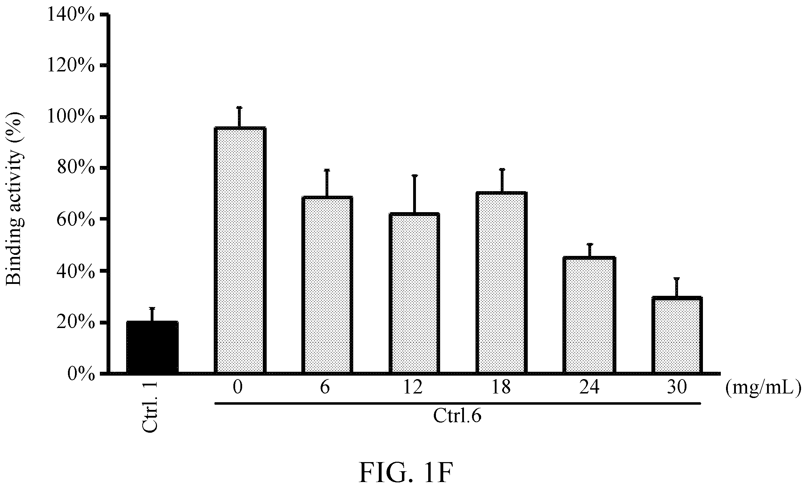

FIGs. 1A to 1G illustrate the bar graphs of the binding activity of viral spike protein to ACE2 receptor inhibited by the herbal compositions of the present disclosure. Ctrl.: No-treatment group; Ctrl. 1: 20 mg/mL of Arbidol; Ctrl. 2: Compound prescription; Ctrl. 3: Lianhua Qingwen capsule; Ctrl. 4: Olive leaf extract; Ctrl. 5: Elderberry extract; Ctrl. 6: Chinese medicine formulation from Hualien Tzu Chi Hospital; Exp. 1 to Exp. 6: the herbal compositions of the present disclosure.

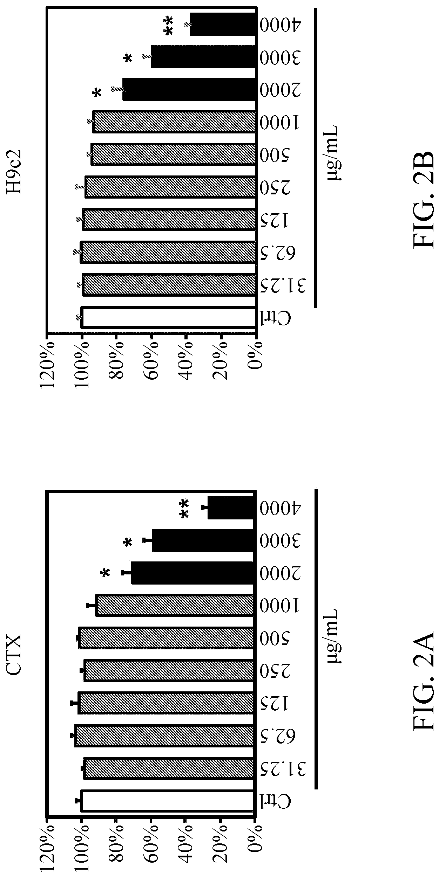

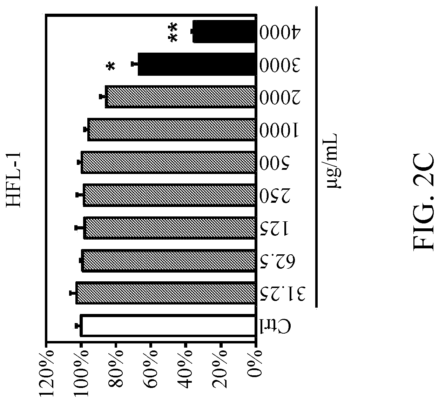

FIGs. 2A to 2C illustrate the bar graphs of cell viability of CTX, H9c2, and HFL-1 treated with the herbal composition of the present disclosure, respectively. Ctrl.: No-treatment group. *: p-value < 0.05, **: p-value < 0.01.

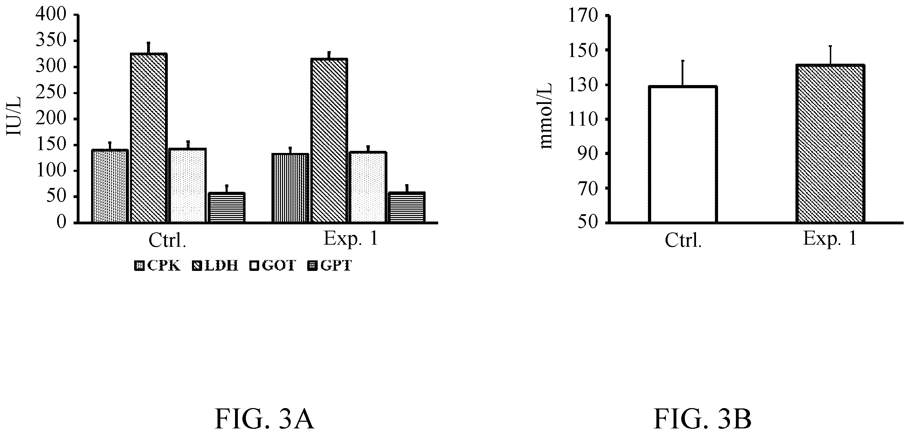

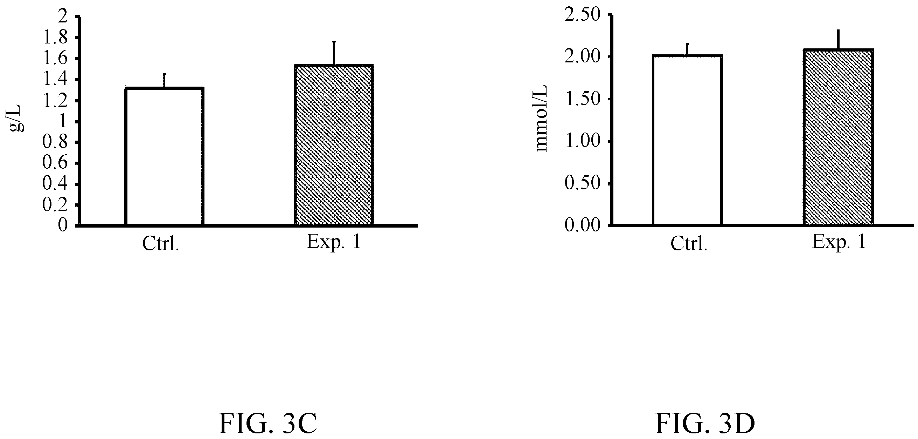

FIGs. 3A to 3D illustrate the bar graphs of the blood parameters in mice treated with the herbal composition of the present disclosure. FIG. 3A shows the levels of creatine phosphokinase (CPK), lactate dehydrogenase (LDH), aspartate transaminase (GOT), and alanine transaminase (GPT) in blood. FIGs. 3B to 3D show the levels of creatinine (CRE), total bilirubin (T-Bil), and glucose (Glu) in blood, respectively. Ctrl.: No-treatment group; Exp. 1: the herbal composition of the present disclosure.

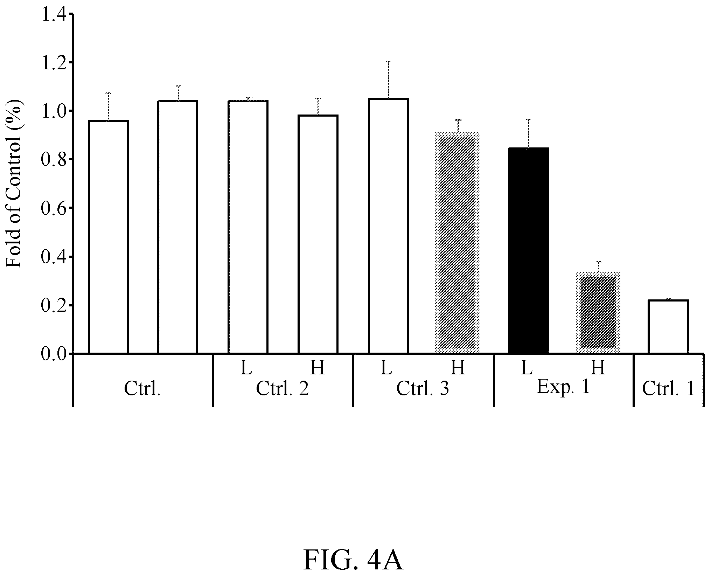

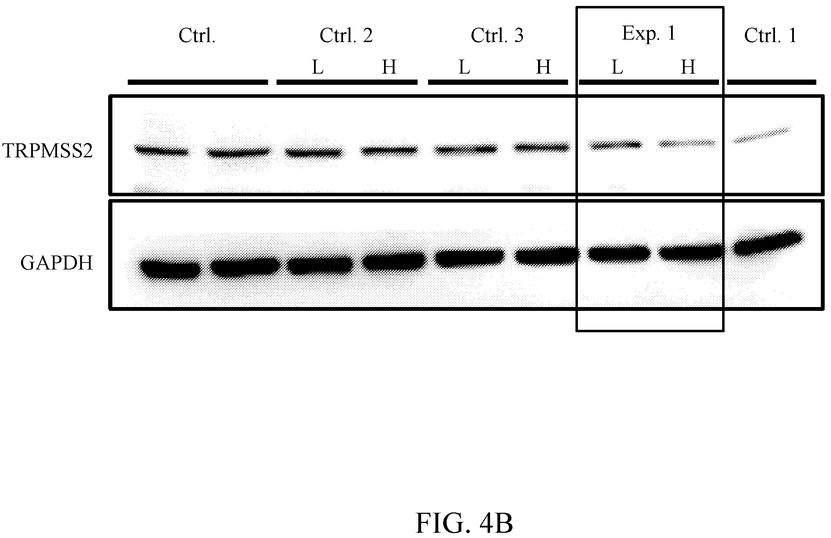

FIGs. 4A and 4B illustrate the expression of transmembrane serine protease 2 (TRPMSS2) analyzed by western blotting. Ctrl.: No-treatment group; Ctrl. 1: 20 mg/mL of Arbidol; Ctrl. 2: Compound prescription; Ctrl. 3: Lianhua Qingwen capsule; Exp. 1: the herbal composition of the present disclosure; L: low dose, 50 µg/ mL; H: high dose, 150 µg/ mL.

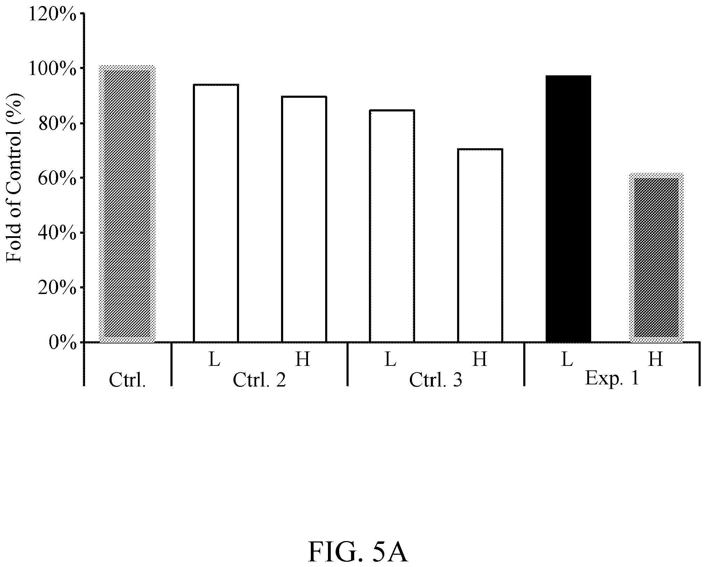

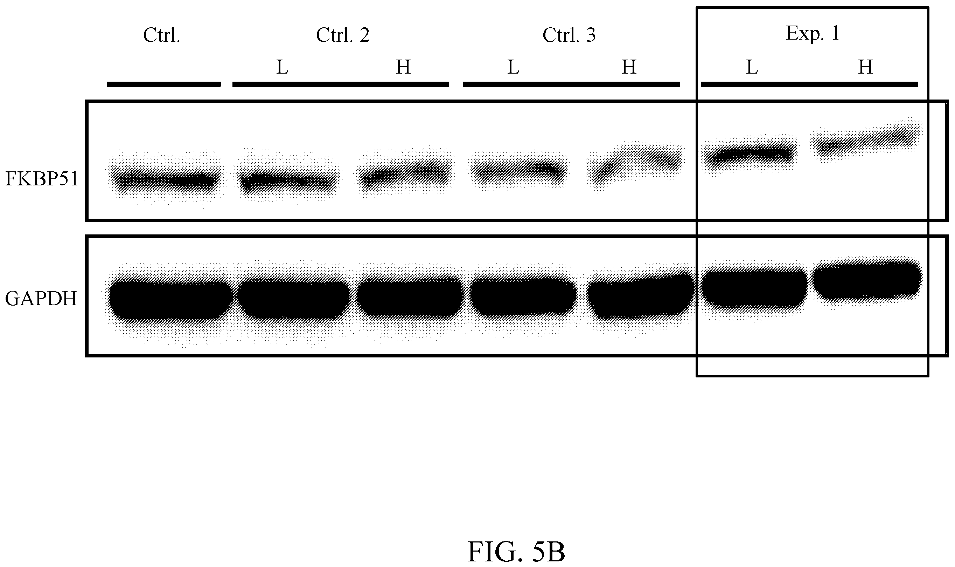

FIGs. 5A and 5B illustrate the expression of FK506-binding protein 51 (FKBP51) analyzed by western blotting. Ctrl.: No-treatment group; Ctrl. 2: Compound prescription; Ctrl. 3: Lianhua Qingwen capsule; Exp. 1: the herbal composition of the present disclosure; L: low dose, 50 µg/ mL; H: high dose, 150 µg/ mL.

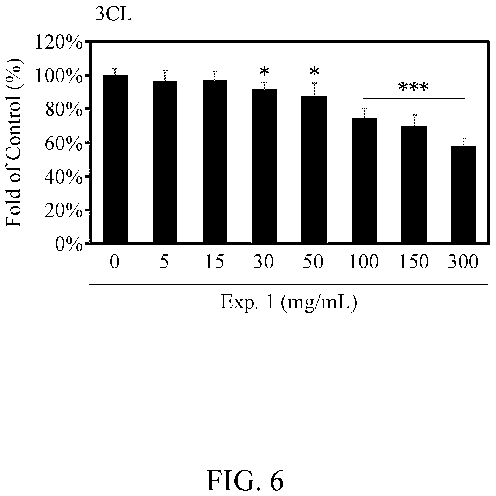

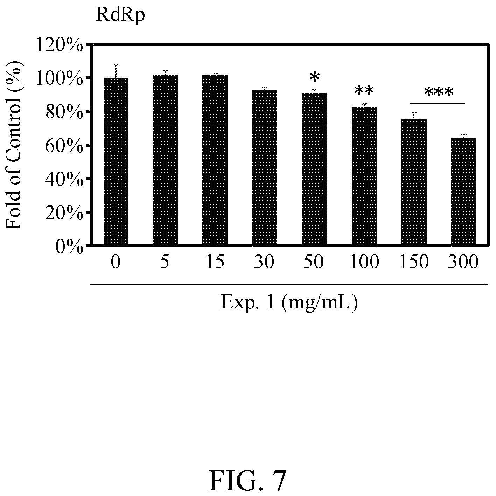

FIGs. 6 and 7 illustrate the bar graphs of the activities of 3CL protease and RNA-dependent RNA polymerase (RdRp) inhibited by the herbal composition of the present disclosure (Exp. 1), respectively. *: p-value < 0.05, **: p-value < 0.01, ***: p-value < 0.001.

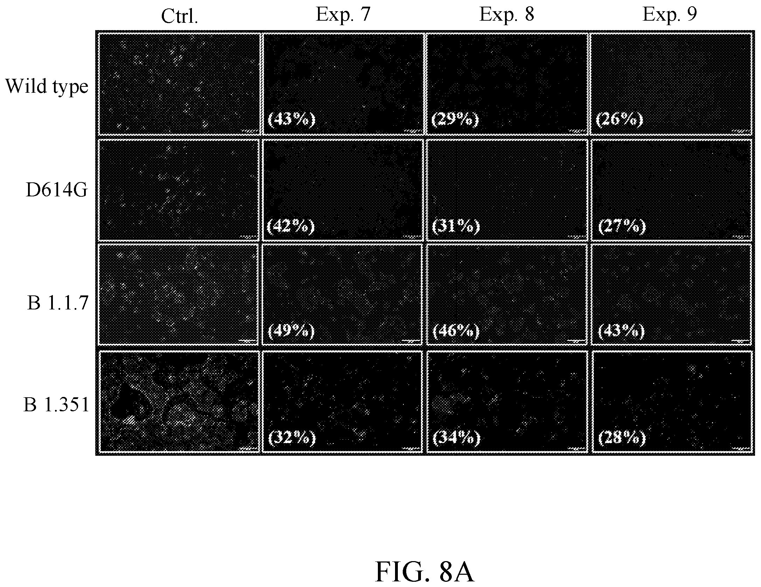

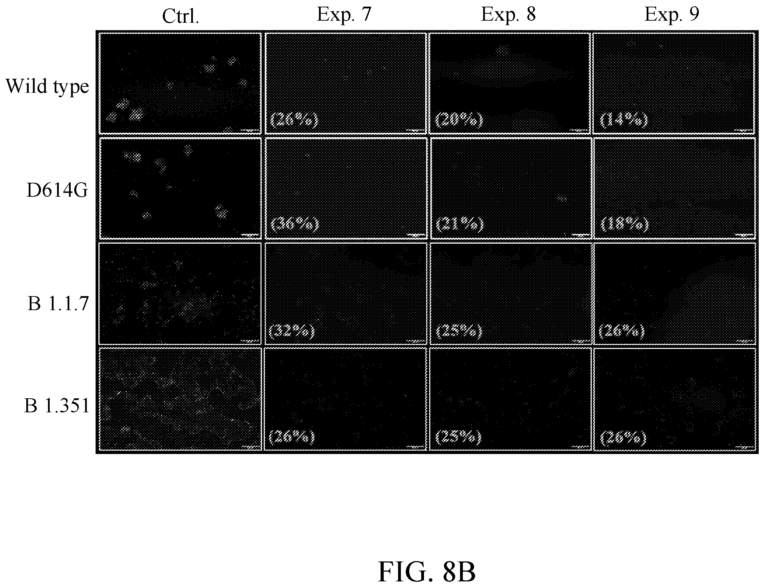

FIGs. 8A and 8B illustrate the inhibitory effect of the herbal composition of the present disclosure against infections of wild type, D614G mutant, B.1.1.7 mutant, and B.1.1351 mutant of SARS-CoV-2 in Caco-2 cells and Calu-3 cells, respectively. Ctrl.: No-treatment group; Exp. 7 to Exp. 9: the herbal compositions of the present disclosure.

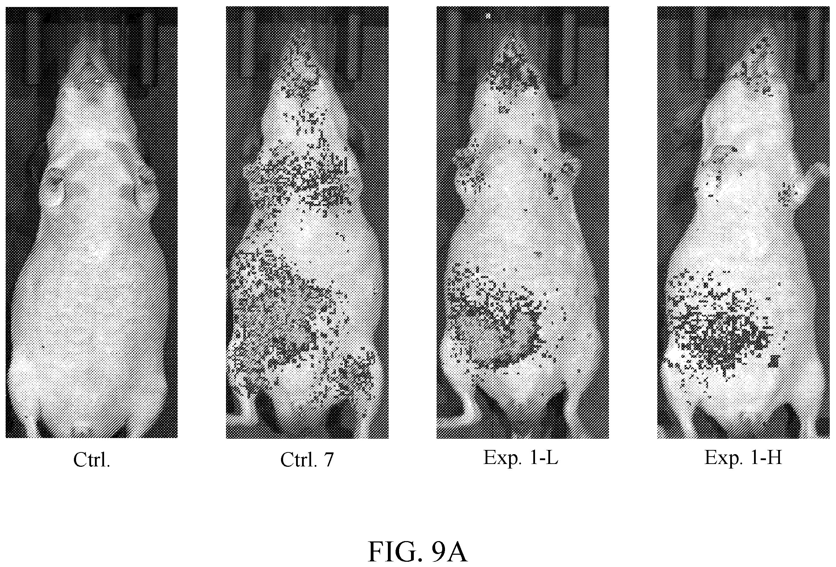

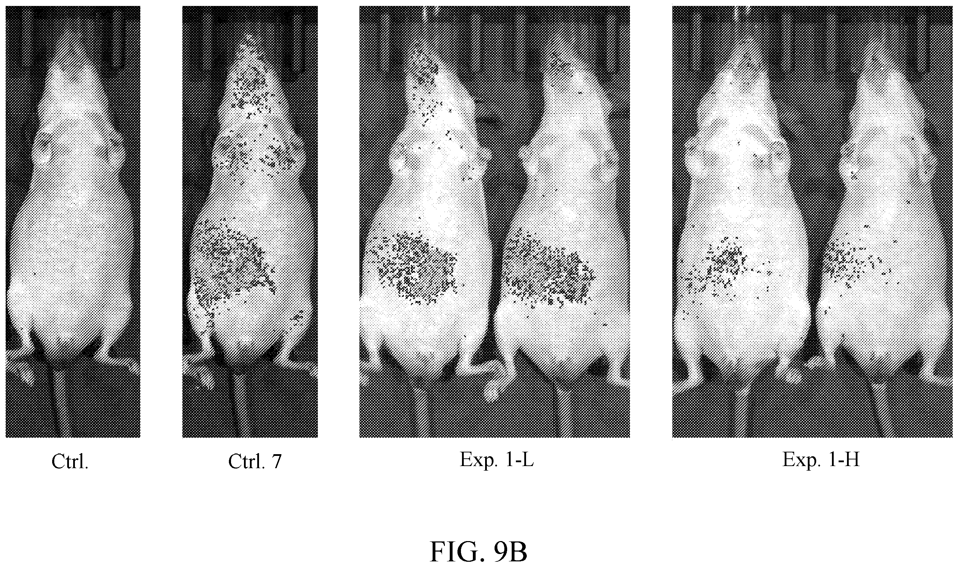

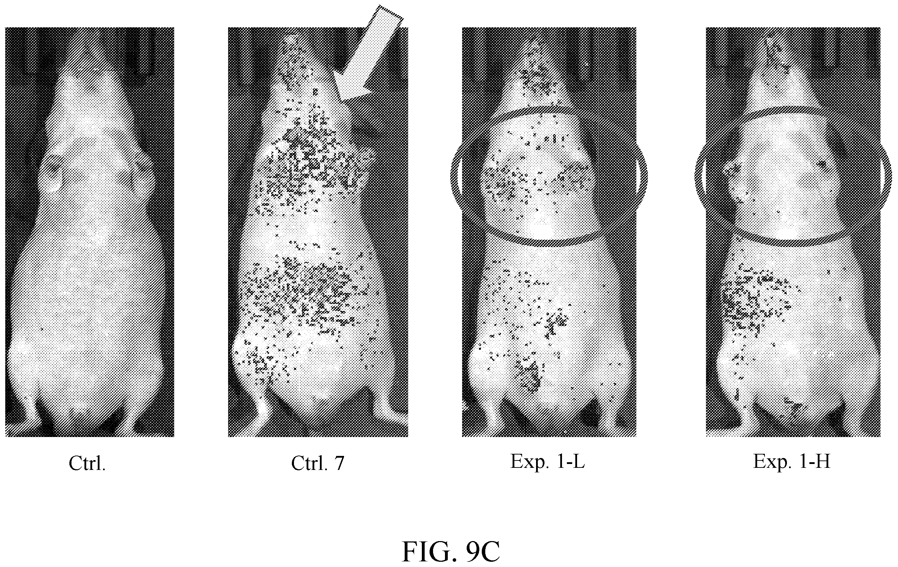

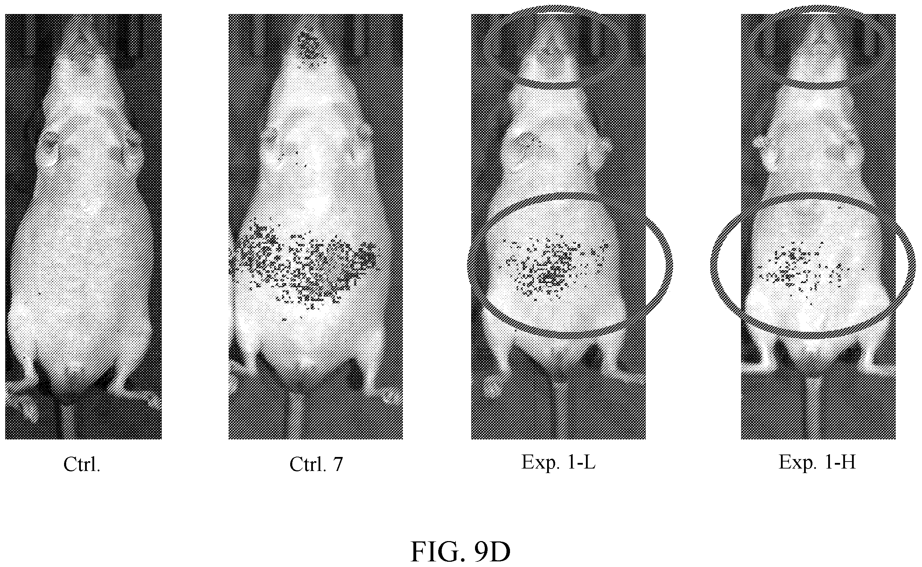

FIGs. 9A to 9D illustrate the inhibitory effect of the herbal composition of the present disclosure against infections of wild type (FIG. 9A), D614G mutant (FIG. 9B), B.1.1.7 mutant (FIG. 9C), and B.1.1351 mutant (FIG. 9D) of SARS-CoV-2 in mice. Ctrl.: No-treatment group; Ctrl.7: Only virus treatment; Exp. 1 - L: low dose of the herbal composition of the present disclosure; Exp. 1 - H: high dose of the herbal composition of the present disclosure.

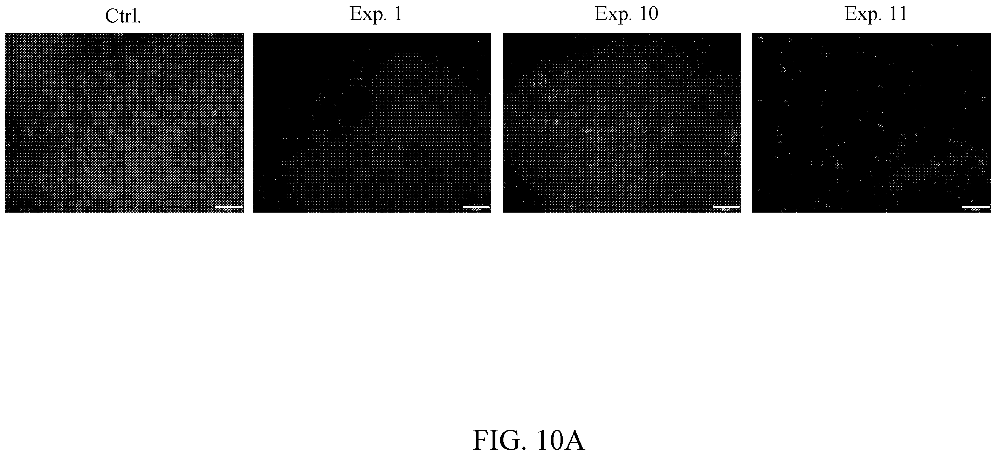

FIG. 10A illustrates the inhibitory effect of the herbal compositions according to different embodiments of the present disclosure against infection of B.1.1.7 mutant of SARS-CoV-2 in Caco-2 cells. Ctrl.: No-treatment group; Exp. 1, Exp. 10, and Exp. 11: the herbal compositions of the present disclosure.

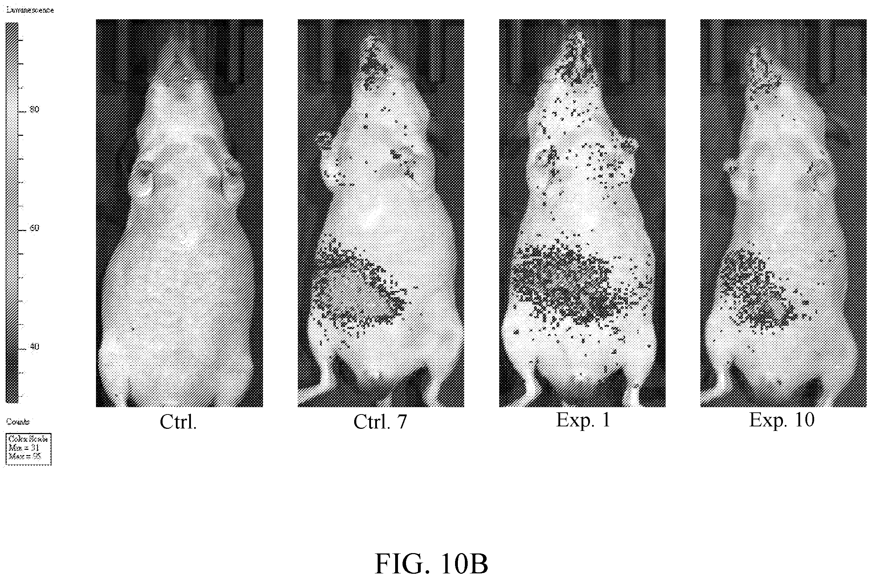

FIG. 10B illustrates the inhibitory effect of the herbal compositions according to different embodiments of the present disclosure against infection of P1 mutant of SARS-CoV-2 in mice. Ctrl.: No-treatment group; Ctrl. 7: Only virus treatment; Exp. 1 and Exp. 10: the herbal compositions of the present disclosure.

### DETAILED DESCRIPTION OF THE EMBODIMENTS

The technical solutions illustrated in the examples of the present disclosure will now be described more clearly and completely, and it will be apparent that the described examples are merely part of the examples of the present disclosure and are not intended to be exhaustive. The present disclosure can also be implemented or applied as described in different examples. All other examples obtained without creative work by those skilled in the art are within the scope of the present disclosure.

It is further noted that, as used in this disclosure, the singular forms "a," "an," and "the" include plural referents unless expressly and unequivocally limited to one referent. The term "or" is used interchangeably with the term "and/or" unless the context clearly indicates otherwise.

As used herein, the term "comprising" or "comprises" is used in reference to compositions, methods, and respective component(s) thereof, which are included in the present disclosure, yet open to the inclusion of unspecified elements or steps, whether essential or not.

The present disclosure is directed to an herbal composition, a method for preparing the herbal composition, and a method for preventing or treating a viral infection in a subject in need thereof by using the herbal composition.

The viral infection treated by the herbal composition of the present disclosure may be caused by coronavirus (CoV).

The structural proteins of CoVs including nucleocapsid (N), small envelope (E), matrix (M) and trimeric spike (S) glycoproteins, which are essential for virion assembly and function to complete the viral life cycle during infections. In some embodiments, the herbal composition may block the interaction of coronavirus S-protein and angiotensin-converting enzyme 2 (ACE2) receptor, as well as suppress the expression of proteins that are necessary for entry and/or replication of the coronavirus in a host, thereby influencing the risk of contracting the viral infection or aggravating the disease progression. Accordingly, the herbal composition of the present disclosure may have antiviral activity and be useful for effectively preventing or treating viral infections caused by a coronavirus.

As used herein, the term "preventing" or "prevention" refers to preventive or avoidance measures for a disease or symptoms or conditions of a disease, which include, but are not limited to, applying or administering one or more active agents to a subj ect who has not yet been diagnosed as a patient suffering from the disease or the symptoms or conditions of the disease but may be susceptible or prone to the disease. The preventive measures of the present disclosure are provided to avoid, prevent, or postpone the occurrence of the disease or the symptoms or conditions of the disease.

As used herein, the term "treating" or "treatment" refers to obtaining a desired pharmacologic and/or physiologic effect, e.g., inhibition of viral entry and/or replication in a host. The effect may be prophylactic in terms of completely or partially preventing a disease or symptoms or conditions thereof, or may be therapeutic in terms of completely or partially curing, alleviating, relieving, remedying, or ameliorating a disease or an adverse effect attributable to the disease or symptoms or conditions thereof.

As used herein, the terms "patient" and "subject" are used interchangeably. The term "subject" means a human or animal. Examples of the subject include, but are not limited to, human, monkey, mice, rat, woodchuck, ferret, rabbit, hamster, cow, horse, pig, deer, dog, cat, fox, wolf, chicken, emu, ostrich, and fish. In some embodiments of the present disclosure, the subject is a mammal, e.g., a primate such as a human.

As used herein, the phrase "an effective amount" refers to the amount of an active agent that is required to confer a desired preventive or therapeutic effect on a subject in need thereof (e.g., reducing the amount of viruses in a host). Effective doses may vary, as recognized by those skilled in the art, depending on routes of administration, excipient usage, the possibility of co-usage with other therapeutic treatment, and the condition to be treated.

As used herein, the term "administering" or "administration" refers to the placement of an active agent into a subject by a method or route which results in at least partial localization of the active agent at a desired site to produce the desired effect. The active agent described herein may be administered by any appropriate route known in the art. For example, the herbal composition of the present disclosure is administered to the subject by oral administration.

The herbal composition of the present disclosure comprises an extract from an herbal raw material and a pharmaceutically acceptable carrier thereof, wherein the herbal raw material comprises *Ohwia caudata.* In some embodiments, the herbal raw material further comprises *Artemisia argyi, Ophiopogon japonicus, Houttuynia cordata, Platycodon grandiflorus, Glycyrrhiza uralensis, Perilla frutescens,* chrysanthemum, or any combination thereof.

In at least one embodiment, the herbal raw material comprises 18% to 25% (e.g., 18%, 19%, 20%, 21%, 22%, 23%, 24%, and 25%) by weight of *Ohwia caudata,* 18% to 25% (e.g., 18%, 19%, 20%, 21%, 22%, 23%, 24%, and 25%) by weight of *Artemisia argyi,* 10% to 17% (e.g., 10%, 11%, 12%, 13%, 14%, 15%, 16%, and 17%) by weight of *Ophiopogon japonicus,* 10% to 17% (e.g., 10%, 11%, 12%, 13%, 14%, 15%, 16%, and 17%) by weight of *Houttuynia cordata,* 10% to 17% by weight of *Platycodon grandiflorus,* 4% to 11% (e.g., 4%, 5%, 6%, 7%, 8%, 9%, 10%, and 11%) by weight of *Glycyrrhiza uralensis,* 4% to 11% (e.g., 4%, 5%, 6%, 7%, 8%, 9%, 10%, and 11%) by weight of *Perilla frutescens,* and 0.4% to 11% (e.g., 0.4%, 0.6%, 0.8%, 1%, 3%, 5%, 7%, 8%, 9%, 10%, and 11%) by weight of chrysanthemum, based on a total weight of the herbal raw material.

In at least one embodiment, the herbal raw material in a range of from around 20 g to around 80 g comprises 5 g to 7 g of *Ohwia caudata,* 5 g to 7 g of *Artemisia argyi,* 3 g to 5 g of *Ophiopogon japonicus,* 3 g to 5 g of *Houttuynia cordata,* 3 g to 5 g of *Platycodon grandiflorus,* 1 g to 3 g of *Glycyrrhiza uralensis,* 1 g to 3 g of *Perilla frutescens,* and 0.1 g to 3 g of chrysanthemum. In some embodiments, the herbal raw material may be in a range of from around 20 g to around 70 g, from around 20 g to around 60 g, from around 20 g to around 50 g, or from around 20 g to around 40 g.

In at least one embodiment, the pharmaceutically acceptable carrier in the herbal composition may be diluents, disintegrants, binders, lubricants, glidants, surfactants, or any combination thereof. The carrier in the composition is "acceptable" in the sense that it is compatible with the active agent of the composition (e.g., capable of stabilizing the active agent) and not deleterious to the subject to be administered. One or more solubilizing agents may be utilized as pharmaceutical excipients for delivery of an active ingredient. Examples of other carriers include colloidal silicon oxide, magnesium stearate, cellulose, and sodium lauryl sulfate. In some embodiments, the herbal composition comprises a pharmaceutically acceptable carrier selected from water, ethanol, maltodextrin, crystalline cellulose, and any combination thereof.

Many examples have been used to illustrate the present disclosure. The examples below should not be taken as a limit to the scope of the present disclosure.

### EXAMPLES

For a more detailed description of the present disclosure, the herbal composition, the preparing method and the use of the composition will be provided and described in detail with reference to the following examples. The materials used in the present disclosure but unannotated herein are commercially available.

### Preparation Example 1-1

6 g of *Artemisia argyi,* 6 g of *Ohwia caudata,* 4 g of *Ophiopogon japonicus,* 4 g of *Houttuynia cordata,* 4 g of *Platycodon grandiflorus,* 2 g of *Glycyrrhiza uralensis,* 2 g of *Perilla frutescens,* and 0.2 g of chrysanthemum were taken and crushed into powder or small pieces. After mixing all the herbal materials with addition of 600 mL water, the mixture was boiled for 5 to 10 minutes. 550 mL of the filtrate was collected by filtration (qualitative filter paper No. 1, TOYO Advantec), and filtered through a 0.22 µm sterile syringe filter, so as to obtain herbal tea. After drying, the amount of the extract of the herbal materials in the obtained herbal tea was measured as about 20 to 40 mg/mL.

### Preparation Example 1-2

6 g of *Artemisia argyi,* 6 g of *Ohwia caudata,* 4 g of *Ophiopogon japonicus,* 4 g of *Houttuynia cordata,* 4 g of *Platycodon grandiflorus,* 2 g of *Glycyrrhiza uralensis,* 2 g of *Perilla frutescens,* and 0.2 g of chrysanthemum were taken and crushed into powder or small pieces. After mixing all the herbal materials with addition of 600 mL water, the mixture was boiled down to approximately 60 mL. The filtrate was collected by filtration (qualitative filter paper No. 1, TOYO Advantec), and filtered through a 0.22 µm sterile syringe filter, so as to obtain herbal tea concentrate. After drying, the amount of the extract of the herbal materials in the obtained herbal tea concentrate was measured as about 150 to 200 mg/mL.

### Preparation Example 2

20 wt% of *Artemisia argyi,* 20 wt% of *Ohwia caudata,* 13.33 wt% of *Ophiopogon japonicus,* 13.33 wt% of *Houttuynia cordata,* 13.33 wt% of *Platycodon grandiflorus,* 6.67 wt% of *Glycyrrhiza uralensis,* 6.67 wt% of *Perilla frutescens,* and 6.67 wt% of chrysanthemum were taken, and the total weight of the herbal materials was about 14 g. Such herbal materials were crushed into small pieces, and packed into a tea bag. The tea bag was immersed in 300 mL of hot water (around 100°C) for 15 to 20 minutes, so as to obtain herbal tea.

### Preparation Example 3 (reference example)

20 wt% of *Artemisia argyi,* 20 wt% of *Ohwia caudata,* 13.33 wt% of *Ophiopogon japonicus,* 13.33 wt% of *Houttuynia cordata,* 13.33 wt% of *Platycodon grandiflorus,* 6.67 wt% of *Glycyrrhiza uralensis,* 6.67 wt% of *Perilla frutescens,* and 6.67 wt% of chrysanthemum were taken, and the total weight of the herbal materials was about 28 g. Such herbal materials were crushed into small pieces, and packed into a tea bag. The tea bag was immersed in 600 mL of 37% alcohol for 30 minutes, followed by boiling for 5 to 10 minutes, so as to obtain an ethanol extract.

### Preparation Example 4

6 g of *Artemisia argyi,* 6 g of *Ohwia caudata,* 4 g of *Ophiopogon japonicus,* 4 g of *Houttuynia cordata,* 4 g of *Platycodon grandiflorus,* 2 g of *Glycyrrhiza uralensis,* 2 g of *Perilla frutescens,* and 0.2 g of chrysanthemum were taken and crushed into powder or small pieces. After mixing all the herbal materials with addition of 600 mL water, the mixture was boiled for 60 minutes. The filtrate was collected by filtration (qualitative filter paper No. 1, TOYO Advantec), filtered through a 0.22 µm sterile syringe filter, and then boiled for concentration to give a water extract (30 mL, about 5 g).

### Preparation Example 5

6 g of *Artemisia argyi,* 6 g of *Ohwia caudata,* 4 g of *Ophiopogon japonicus,* 4 g of *Houttuynia cordata,* 4 g of *Platycodon grandiflorus,* 2 g of *Glycyrrhiza uralensis,* 2 g of *Perilla frutescens,* and 0.2 g of chrysanthemum were taken and crushed into powder or small pieces. After mixing all the herbal materials with addition of 225.6 mL water (8 times of the total weight of the herbal materials), the mixture was boiled for 60 minutes, and then filtered to obtain the first filtrate. After adding 141.0 mL of water (5 times of the total weight of the herbal materials) to the residue, the mixture was boiled for further 60 minutes, and then filtered to obtain the second filtrate. The first and second filtrates were combined and filtered through a 0.22 µm sterile syringe filter, and then boiled for concentration to give a water extract (36.66 mL, about 5 g).

### Preparation Example 6

In this example, a water extract was prepared by the process described in Preparation Example 4 or 5, except that the leaves of *Ohwia caudate* used in Preparation Example 4 or 5 were replaced with the roots of *Ohwia caudate* in this example.

### Preparation Example 7 (reference example)

In this example, a water extract was prepared by the process described in Preparation Example 4 or 5, except that *Ohwia caudate* was replaced with *Anisomeles indica* (L.) O. Ktze.

### Preparation Example 8 (reference example)

In this example, a water extract of *Artemisia argyi* was prepared by the process described in Preparation Example 4 or 5, except that the herbal material used in this example was 6 g of *Artemisia argyi* alone.

### Preparation Example 9

In this example, a water extract of *Ohwia caudata* was prepared by the process described in Preparation Example 4 or 5, except that the herbal material used in this example was 6 g of *Ohwia caudata* alone.

### Pharmacological Example 1: Materials and Methods

The therapeutic effect of the herbal composition provided in the present disclosure for the prevention or treatment of coronavirus infection was determined in the following Pharmacological Examples 2 to 7. The samples to be tested were obtained from Preparation Examples 1 to 9, and the comparative samples as control groups were listed as follows:
Control group (Ctrl.) - No-treatment group;
Control group 1 (Ctrl. 1) - Arbidol, also known as Umifenovir, which is a Russian-made broad-spectrum antiviral drug used for some enveloped and non-enveloped viruses;
Control group 2 (Ctrl. 2) - Compound prescription, comprising lactoferrin, colloidal silver, olive leaf extract, and elderberry extract;
Control group 3 (Ctrl. 3) - Lianhua Qingwen capsule, which is a traditional Chinese medicine formulation used for the treatment of influenza, and comprises *Forsythia suspensa, Lonicera japonica, Ephedra sinica,* bitter almond, gypsum, roots of *Isatis indigotica, Dryopteris crassirhizoma, Houttuynia cordata, Pogostemon cablin,* rhubarb, *Rhodiola rosea,* menthol and *Glycyrrhiza uralensis;*
Control group 4 (Ctrl. 4) - Olive leaf extract;
Control group 5 (Ctrl. 5) - Elderberry extract; and
Control group 6 (Ctrl. 6) - Chinese medicine formulation from Hualien Tzu Chi Hospital (Taiwan), comprising *Ophiopogon japonicus, Houttuynia cordata, Platycodon grandiflorus,* chrysanthemum, *Glycyrrhiza uralensis,* and *Perilla frutescens.*

Further, the experimental methods used in these examples were described as follows:

### (1) Assay of inhibition of viral spike protein binding to angiotensin-converting enzyme 2 (ACE2) receptor

The effect of the test samples for inhibition of SARS-CoV-2 spike protein binding to human ACE2 receptor was determined by using the COVID-19 Spike-ACE2 Binding Assay Kit II (RayBiotech) according to the manufacturer's instructions.

Briefly, all reagents were brought to room temperature (about 18°C to 25°C) before use. Next, 100 µL of each test sample was added into the well of removable 8-well strips, and covered with the plate sealing film and incubated for 2.5 hours at room temperature or overnight at 4°C with gentle shaking. Then, the solution in wells was discarded, and each well was washed 4 times with 1 × Wash Solution. Further, each well was washed by filling with 1 × Wash Buffer (300 µL) using a multi-channel pipette or autowasher. After the last wash, the remaining 1× Wash Buffer was removed by aspirating or decanting, and 100 µL of 1× mouse secondary horseradish peroxidase (HRP)-conjugated IgG was added to each well and incubated for 1 hour at room temperature with gentle shaking. After that, the solution in wells was discarded, and each well was washed as described above. Subsequently, 100 µL of 3,3',5,5'-tetramethylbenzidine (TMB) One-Step Substrate Reagent was added to each well, and incubated for 30 minutes at room temperature in the dark with gentle shaking. Finally, 50 µL of Stop Solution was added to each well, and the optical absorbance at 450 nm was read immediately.

### (2) Assay of inhibition of 3C-like (3CL) protease of virus

The effect of the test samples for inhibition of 3CL protease of SARS-CoV-2 to resist viral replication in human cells was determined by using SensoLyte SARS-CoV-2 3CL Protease Activity Assay Kit (Fluorimetric) according to the manufacturer's instructions.

Briefly, the working solutions were prepared firstly. Specifically, 1 × assay buffer was prepared by adding 10 mL of 2× assay buffer to 10 mL of deionized water. The 3CL protease substrate solution was prepared by diluting the 3CL protease substrate 100-fold with the assay buffer. The 3CL protease diluent was prepared by diluting the 3CL protease 80-fold with the assay buffer. The inhibitor (GC 376) diluent was prepared by diluting the 10 µM inhibitor solution 100-fold with the assay buffer.

Next, for the enzymatic reaction, the test samples and the 3CL protease diluent were added to the microplate wells in an amount of 10 µL/well and 40 µL/well, respectively. Simultaneously, the following control wells were set up: Positive control, containing 3CL protease without the test sample; Inhibitor control, containing 3CL protease and inhibitor GC 376; Vehicle control, containing 3CL protease and vehicle used in delivering the test sample (e.g., dimethyl sulfoxide (DMSO) with concentration not exceeding 1%); Test sample control, containing assay buffer and test sample; and Substrate control, containing assay buffer. The total volume of all controls was brought to 50 µL by using the assay buffer.

Subsequently, 50 µL of the 3CL protease substrate solution was added into each well, and then the fluorescence signal was measured by kinetic reading or end-point reading. For kinetic reading, the fluorescence intensity was measured immediately at excitation/emission (Ex/Em) = 490 nm/520 nm continuously, and the data were recorded every 5 minutes for 30 to 60 minutes. For end-point reading, the reaction solution was incubated at 37°C for 30 to 60 minutes and kept from direct light, and then the fluorescence intensity was measured at Ex/Em = 490 nm/520 nm.

### (3) Assay of inhibition of RNA-dependent RNA polymerase (RdRp) of virus

The effect of the test samples for inhibition of RNA polymerase of SARS-CoV-2 to resist viral replication in human cells was determined by using SARS-CoV-2 RNA Polymerase (RdRp) Assay Kit (ProFoldin) according to the manufacturer's instructions.

Briefly, the SARS-CoV2 RdRp assay was performed in 96-well plate format. Firstly, 1 µL of the test sample in DMSO was added into each well of the 96-well assay plate. Further, 48 µL of a premix composed of 41 µL of H₂O, 5 µL of 10× buffer, 1 µL of 50× template, and 1 µL of 50× RdRp was added, followed by incubating for 5 min. Subsequently, 1 µL of 50× NTP was added and further incubated at 34°Cfor 60 to 120 minutes. After reaction, 150 µL of 1× fluorescence dye solution was added to the incubated reaction mixture, and the fluorescence intensity was measured at 450 nm in 5 minutes.

### (4) Assay of inhibition of transmembrane serine protease 2 (TRPMSS2) and FK506-binding protein 51 (FKBP51)

The effects of the test samples for inhibition of TRPMSS2 to resist the attachment and invasion of SARS-CoV-2 into host cells and for reduction of FKBP51 to resist the stress responses were determined by culturing 4×10⁵ Caco-2 cells in a 10 cm petri dish for 24 hours, adding the test samples in a concentration of 50 µg/mL (low dose) or 150 µg/mL (high dose), and after 12 hours, collecting the cells and analyzing the levels of TRPMSS2 and FKBP51 by western blotting.

### (5) SARS-CoV-2 pseudotyped lentivirus assay

The SARS-CoV-2 pseudotyped lentivirus was provided by the RNAi Core of Academia Sinica (Taiwan), which was a lentivirus having a green fluorescent protein gene or luciferase gene in its genome and expressing SARS-CoV-2 spike protein on its surface envelope. For determining the effect of the test samples for prevention of SARS-CoV-2 infection *in vitro,* the human intestinal epithelial cell line Caco-2 and human lung cell line Calu-3 were cultured for 12 hours, and then co-cultured with 10 µg/mL or 30 µg/mL of the test samples for 24 hours. Afterward, the cultured cells were infected with wild type, D614G mutant, Alpha mutant (B.1.1.7), or Beta mutant (B.1.351) of the SARS-CoV-2 pseudotyped lentivirus. After 24 hours, the infected cells were observed by fluorescence microscope.

On the other hand, for the *in vivo* assay, the SKH2/J mice were treated with the test samples by gavage (16.22 mg or 48.66 mg/0.3 mL/mouse/day) for seven consecutive days. On day 4 to day 6, the mice were infected with wild type, D614G mutant, Alpha mutant (B.1.1.7), Beta mutant (B.1.351), or P1 mutant (Brazil variant) of the SARS-CoV-2 pseudotyped lentivirus by intranasal delivery with Aerogen Solo nebulizer (10 µL of 1.2×10⁶ particles/mouse/day). On day 8, the viral infection in mice was determined by *in vivo* imaging system (IVIS).

The parameters of Aerogen Solo nebulizer were as follows. Flow rate: greater than 0.2 mL/min (average: about 0.38 mL/min); Particle size: (1) specification range: 1 µm to 5 µm, average value: 3.1 µm, measured by Andersen cascade impact sampler; (2) specification range: 1.5 µm to 6.2 µm, average value: 3.9 µm, measured by Marple 298 cascade impact sampler. According to Standard EN 13544-1, the starting dose was 2 mL, and the aerosol output speed was 0.30 mL/min. Further, the aerosol output was 1.02 mL/dose, and the residual volume was less than 0.1 mL per 3 mL dose.

### (6) Cytotoxicity and cell viability assay

For determining the toxicity of the test samples *in vitro,* MTT (3-(4,5-dimethylthiazol-2-yl)-2,5-diphenyltetrazolium bromide) assay was performed to evaluate the cell activity and proliferation. Specifically, astrocytes CTX, myocardial cells H9c2, and lung fibroblast HFL-1 were co-cultured with the test samples in a concentration of from 31.25 µg/mL to 4,000 µg/mL for 24 hours. Afterward, MTT was added to the culture and incubated for 2 hours. By dissolving the MTT crystals in dimethyl sulfoxide (DMSO), the optical absorbance at 590 nm was measured by an enzyme-linked immunosorbent assay (ELISA) reader.

On the other hand, for determining the toxicity of the test samples *in vivo,* the C57BL/6 mice were treated with the test samples by gavage at a dose of 5,200 mg/kg/day. After 24 hours, the levels of creatine phosphokinase, lactate dehydrogenase, aspartate transaminase, alanine transaminase, creatinine, total bilirubin, and glucose in blood of mice were measured.

### Pharmacological Example 2: Inhibition of viral spike protein binding to ACE2 receptor

The effect of the herbal composition prepared from Preparation Example 4 or 5 (Exp. 1, 6 to 30 mg/mL) for inhibition of SARS-CoV-2 spike protein binding to human ACE2 receptor was determined and compared with that of Arbidol (Ctrl. 1, 20 mg/mL), Compound prescription (Ctrl. 2), Lianhua Qingwen capsule (Ctrl. 3), olive leaf extract (Ctrl. 4), elderberry extract (Ctrl. 5), and Chinese medicine formulation from Hualien Tzu Chi Hospital (Ctrl. 6).

The results were shown in FIGs. 1A to 1F, indicating that as compared with Ctrl. 2 to Ctrl. 6, Exp. 1 exhibited greatly improved blocking activity for the viral spike protein binding to ACE2 receptor. In addition, referring to FIGs. 1B and 1C, the binding activities of the spike protein treated with 6 mg/mL and 12 mg/mL of Exp. 1 were comparable to that treated with 24 mg/mL and 30 mg/mL of Ctrl. 3, respectively; that is to say, under the same administered dose, the blocking activity of the herbal composition of the present application was about 2.5 to 4 times higher than Lianhua Qingwen capsule. Furthermore, FIG. 1C showed that 18 mg/mL of Exp. 1 may achieve a higher blocking effect than 20 mg/mL of Arbidol, implying that a low dose of the herbal composition of the present disclosure may effectively block the interaction between the viral spike protein and ACE2 receptor, such that the severe side effects such as nausea, diarrhea, and dizziness caused by the administration of conventional antiviral drugs (e.g., Arbidol) can be avoided.

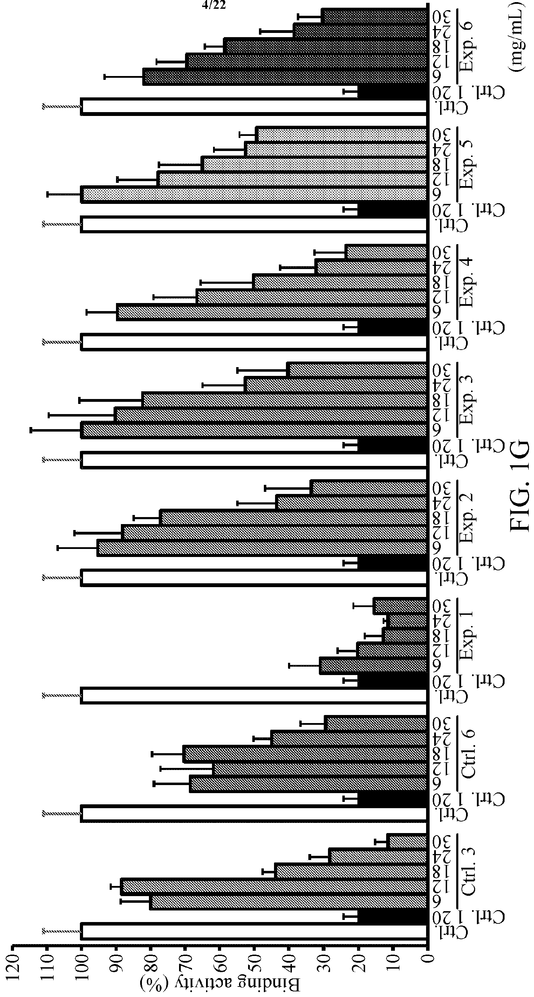

In addition, FIG. 1G showed the comparison of the herbal compositions prepared by different extraction methods in view of the effect for inhibition of SARS-CoV-2 spike protein binding to human ACE2 receptor, in which the water extract prepared from Preparation Example 4 or 5 was notated as Exp. 1; the herbal tea prepared from Preparation Example 1 was notated as Exp. 2; the herbal tea prepared from Preparation Example 2 was notated as Exp. 3; the ethanol extract prepared from Preparation Example 3 was notated as Exp. 4; the extract of *Artemisia argyi* prepared from Preparation Example 8 was notated as Exp. 5; and the extract of *Ohwia caudate* prepared from Preparation Example 9 was notated as Exp. 6. These results demonstrated that the herbal compositions of the present disclosure prepared by different extraction methods may also block the interaction between the viral spike protein and ACE2 receptor.

### Pharmacological Example 3: Safety of the herbal composition of the present disclosure

The toxicity of the herbal composition prepared from Preparation Example 4 or 5 (Exp. 1) was determined by MTT assay and animal model.

The results of MTT assay of cell lines CTX, H9c2, and HFL-1 were shown in FIGs. 2A to 2C, respectively, illustrating that the herbal composition of the present disclosure in an amount of less than 1,000 µg/mL would not have impact on the physiological activities of brain, heart and lung. Since the herbal composition of the present disclosure as the drinkable herbal tea provided in Preparation Example 1-1 comprises the extract of the herbal raw materials in an amount of 20 to 40 mg/mL, it is implied that the herbal composition of the present disclosure would not cause toxicity even though a 70 kg adult human swigged 5.6 L of the herbal composition.

In the animal model, C57BL/6 mice were fed with the herbal composition of the present disclosure at a dose of 5,200 mg/kg/mouse/day, which is 20 times of the recommended daily intake for a person. As a result, the levels of creatine phosphokinase (CPK), lactate dehydrogenase (LDH), aspartate transaminase (GOT), alanine transaminase (GPT), creatinine (CRE), total bilirubin (T-Bil), and glucose (Glu) in blood of mice were shown in Table 1 below and FIGs. 3A and 3B. CPK and LDH are indicators of heart and brain functions; GOT and GPT are indicators of liver function; CRE is an indicator of kidney function; and T-Bil includes direct bilirubin and indirect bilirubin, which are indicators of gallbladder function. These results indicated that there is no significant difference in the blood parameters between the mice treated with and without the herbal composition, implying the herbal composition of the present disclosure would not result in an adverse effect even in an extremely high dose.

**Table 1. Blood parameters of mice after administration of the herbal composition of the present disclosure**

| Indicator | Ctrl. | Exp.1 | Unit |
|---|---|---|---|
| CPK | 126.73 | 122.77 | IU/L |
| LDH | 316.37 | 311.87 | IU/L |
| GOT | 137.03 | 130.70 | IU/L |
| GPT | 53.27 | 55.83 | IU/L |
| CRE | 134.17 | 137.03 | mmol/L |
| T-Bil | 1.35 | 1.46 | g/L |
| Glu | 2.06 | 2.01 | mmol/L |

### Pharmacological Example 4: Inhibition of expressions of TRPMSS2 and FKBP51

The effect of the herbal composition prepared from Preparation Example 4 or 5 (Exp. 1) for inhibition of expressions of TRPMSS2 and FKBP51 was determined and compared with that of Arbidol (Ctrl. 1, 20 mg/mL), Compound prescription (Ctrl. 2), and Lianhua Qingwen capsule (Ctrl. 3).

The results of inhibition of TRPMSS2 expression were shown in FIGs. 4A and 4B, indicating that Exp.1 significantly inhibited TRPMSS2 expression in comparison with Ctrl. 2 and Ctrl. 3. Moreover, when the administrated dose of Exp.1 was increased from 50 µg/mL (low dose, L) to 150 µg/mL (high dose, H), the TRPMSS2 expression was decreased by 70%, whereas when the administrated dose of Ctrl. 3 was increased from 50 µg/mL (low dose, L) to 150 µg/mL (high dose, H), the TRPMSS2 expression was only decreased by 10%. That is to say, the herbal composition of the present disclosure may inhibit TRPMSS2 expression with seven times better efficacy in comparison with Lianhua Qingwen capsule, implying the potent effect of the herbal composition of the present disclosure for blocking viral entry into the host cell.

The results of inhibition of FKBP51 expression were shown in FIGs. 5A and 5B, indicating that when the administrated dose of Exp.1 was increased from 50 µg/mL (low dose, L) to 150 µg/mL (high dose, H), the FKBP51 expression was decreased by 40%. FKBP51 is a molecular link to stress, such that these results implied that the herbal composition of the present disclosure has an effect against emotional stress, anxiety and depression which may be caused by COVID-19.

### Pharmacological Example 5: Inhibition of activities of 3CL protease and RdRp

The effect of the herbal composition prepared from Preparation Example 4 or 5 (Exp. 1) for inhibition of activities of 3CL protease and RdRp of SARS-CoV-2 was determined.

The results were shown in FIG. 6 (3CL) and FIG. 7 (RdRp), respectively, indicating that the herbal composition of the present disclosure reduced the activities of RdRp and 3CL protease by 35% to 40% in a dose-dependent manner. For SARS-CoV-2, the 3CL involves in the cleavage of polyproteins, giving rise to viral proteins essential for the life cycle of the virus, and RdRp is used for the replication of viral genome and the transcription of viral genes. Accordingly, these results implied that the herbal composition of the present disclosure has an effect on the treatment of COVID-19.

### Pharmacological Example 6: Prevention of SARS-CoV-2 infection

The effect of the herbal composition of the present disclosure for prevention of SARS-CoV-2 infection was determined by using wild type and variants of SARS-CoV-2 pseudotyped lentivirus.

For the *in vitro* assay, the herbal compositions to be tested were as follows: Exp. 7, containing 1.5 g of the water extract prepared from Preparation Example 4 or 5 and 1.5 g of excipient (a mixture of maltodextrin and crystalline cellulose) (the concentration of the water extract: 10 µg/mL); Exp. 8, containing 3 g of the water extract prepared from Preparation Example 4 or 5 and 3 g of excipient (a mixture of maltodextrin and crystalline cellulose) (the concentration of the water extract: 30 µg/mL); and Exp. 9, containing 3 g of the water extract prepared from Preparation Example 4 or 5 (the concentration of the water extract: 30 µg/mL). Each composition was added to Caco-2 cells or Calu-3 cells. After 24 hours, the wild type pseudotyped lentivirus and three variants thereof (i.e., D614G mutant, B.1.1.7 mutant, and B.1.351 mutant) were added.

The results were shown in FIG. 8A (Caco-2) and FIG. 8B (Calu-3), indicating that the herbal composition of the present disclosure may reduce viral infection of both wild type and variants by 51% to 74 % for Caco-2 and by 64% to 86 % for Calu-3.

For the *in vivo* assay, the herbal composition prepared from Preparation Example 4 or 5 was administrated to SKH2/J mice by gavage at a dose of 16.22 mg/0.3 mL/mouse/day (Exp. 1 - L) or 48.66 mg/0.3 mL/mouse/day (Exp. 1 - H) for seven consecutive days, and the wild type pseudotyped lentivirus and three variants thereof (i.e., D614G mutant, B.1.1.7 mutant, and B.1.351 mutant) were administrated to the mice by intranasal delivery on day 4 to day 6.

The results were shown in FIG. 9A (wild type), FIG. 9B (D614G mutant), FIG. 9C (B.1.1.7 mutant) and FIG. 9D (B.1.351 mutant), in which Ctrl. was the group of mice without any drug or virus treatment, and Ctrl. 7 was the group of mice only treated with the pseudotyped lentivirus. As shown in FIG. 9A, the mice treated with the herbal composition of the present disclosure exhibited lower luminance levels in their tissues, implying that the herbal composition of the present disclosure may effectively prevent infection of wild type SARS-CoV-2.

Further, as shown in FIG. 9B, the infection of the D614G mutant in the mice was reduced by 5 times and 25 times in Exp. 1 - L and Exp. 1 - H, respectively, implying that the herbal composition of the present disclosure may effectively prevent infection of the D614G mutant.

As shown in FIG. 9C, the mice of Ctrl. 7 had severe lung infection caused by the B.1.1.7 mutant (as indicated by the arrow), while Exp. 1 - L and Exp. 1 - H may effectively prevent the B.1.1.7 mutant from strongly infecting the lungs (as indicated by circle areas).

As shown in FIG. 9D, the infection of the B.1.351 mutant was reduced in Exp. 1 - L and Exp. 1 - H; in particular, as indicated by the circle areas, the nasal and intestinal infection caused by the B.1.351 mutant was significantly reduced in Exp. 1 - L and Exp. 1 - H.

These above results indicated that the herbal composition of the present disclosure may significantly prevent viral infection of both wild type and mutant variants.

### Pharmacological Example 7: The effect of the herbal compositions of the present disclosure containing replaceable components

In this example, the effect of the herbal composition prepared from Preparation Example 4 or 5 (i.e., those containing the extract of *Ohwia caudate* leaves) for prevention of SARS-CoV-2 infection was compared to that prepared from Preparation Example 6 (i.e., those containing the extract of *Ohwia caudate* roots). Also, since *Ohwia caudate* and *Anisomeles indica* (L.) O. Ktze may generally be identified as the same Chinese medicinal material, the effect of the herbal composition prepared from Preparation Example 4 or 5 was also compared to that prepared from Preparation Example 7 (i.e., those containing the extract of *Anisomeles indica* (L.) O. Ktze). The comparison was performed by determining the effect of these herbal compositions for preventing the infection of SARS-CoV-2 pseudotyped lentivirus.

The results were shown in FIGs. 10A and 10B, in which the herbal composition containing the extract of *Ohwia caudate* leaves was notated as Exp. 1; the herbal composition containing the extract of *Ohwia caudate* roots was notated as Exp. 10; the herbal composition containing the extract of *Anisomeles indica* (L.) O. Ktze was notated as Exp. 11; and Ctrl. 7 was the group of mice only treated with the P1 mutant of SARS-CoV-2 pseudotyped lentivirus.

In FIG. 10A, the B.1.1.7 mutant of SARS-CoV-2 pseudotyped lentivirus was used to infect Caco-2 cells treated with 30 µL/mL of the test sample, and the results showed that like Exp. 1, Exp. 10 and Exp. 11 may also reduce viral infection by about 48% and about 64%, respectively.

In FIG. 10B, the SKH2/J mice were fed with the test sample at a dose of 48.66 mg/0.3 mL/mouse/day before the infection of the P1 mutant, and the results showed that the infection of the P1 mutant in the mice was reduced by 25 times and 40 times in Exp. 1 and Exp. 10, respectively.

These above results indicated that the herbal composition containing the extract of *Ohwia caudate* roots or the extract of *Anisomeles indica* (L.) O. Ktze may also significantly prevent SARS-CoV-2 infection.

## Claims

1. An herbal composition for use in preventing or treating a viral infection caused by a coronavirus, comprising an extract from an herbal raw material and a pharmaceutically acceptable carrier, wherein the herbal raw material comprises *Ohwia caudata,* wherein the *Ohwia caudata* is an *Ohwia caudata* root, or a combination of the *Ohwia caudata* root and an *Ohwia caudata* leaf, and wherein the extract from the herbal raw material is a water extract.

2. The herbal composition for use according to claim 1, wherein the herbal raw material further comprises at least one of *Artemisia argyi, Ophiopogon japonicus, Houttuynia cordata, Platycodon grandiflorus, Glycyrrhiza uralensis, Perilla frutescens,* and chrysanthemum.

3. The herbal composition for use according to claim 2, wherein the herbal raw material comprises, based on a total weight thereof, 18% to 25% by weight of *Ohwia caudata,* and at least one of 18% to 25% by weight of *Artemisia argyi,* 10% to 17% by weight of *Ophiopogon japonicus,* 10% to 17% by weight of *Houttuynia cordata,* 10% to 17% by weight of *Platycodon grandiflorus,* 4% to 11% by weight of *Glycyrrhiza uralensis,* 4% to 11% by weight of *Perilla frutescens,* and 0.4% to 11% by weight of chrysanthemum, and/or wherein the herbal raw material comprises 5 g to 7 g of *Ohwia caudata,* and at least one of 5 g to 7 g of *Artemisia argyi,* 3 g to 5 g of *Ophiopogon japonicus,* 3 g to 5 g of *Houttuynia cordata,* 3 g to 5 g of *Platycodon grandiflorus,* 1 g to 3 g of *Glycyrrhiza uralensis,* 1 g to 3 g of *Perilla frutescens,* and 0.1 g to 3 g of chrysanthemum.

4. The herbal composition for use according to claim 1, wherein the coronavirus is severe acute respiratory syndrome coronavirus (SARS-CoV), Middle East respiratory syndrome coronavirus (MERS-CoV), SARS-CoV-2, mouse hepatitis virus (MHV), or porcine epidemic diarrhea virus (PEDV).

5. The herbal composition for use according to claim 4, wherein the coronavirus is a variant of SARS-CoV-2.

6. The herbal composition for use according to claim 1, wherein the extract from the herbal raw material in the herbal composition is administered to the subject in an effective amount of from about 25 mg/kg/day to about 2,500 mg/kg/day.

7. The herbal composition for use according to claim 6, wherein the extract from the herbal raw material in the herbal composition is administered to the subject in an effective amount of from about 50 mg/kg/day to about 500 mg/kg/day.

8. The herbal composition for use according to claim 1, wherein the herbal composition interferes with binding of a spike protein of a virus to an angiotensin-converting enzyme 2 (ACE2) receptor.

9. The herbal composition for use according to claim 1, wherein the herbal composition suppresses expression of at least one of 3CL protease, RNA-dependent RNA polymerase (RdRp), transmembrane serine protease 2 (TRPMSS2), and FK506 binding protein 5 (FKBP5).

## Patentansprüche

1. Pflanzliche Zusammensetzung zur Verwendung bei der Vorbeugung oder Behandlung einer durch ein Coronavirus verursachten Virusinfektion, umfassend einen Extrakt aus einem pflanzlichen Rohmaterial und einen pharmazeutisch verträglichen Träger, wobei das pflanzliche Rohmaterial Ohwia caudata umfasst, wobei es sich bei *Ohwia caudata* um eine Wurzel von *Ohwia caudata* oder eine Kombination aus der Wurzel von *Ohwia caudata* und einem Blatt von *Ohwia caudata* handelt und wobei der Extrakt aus dem pflanzlichen Rohmaterial ein Wasserextrakt ist.

2. Pflanzliche Zusammensetzung zur Verwendung nach Anspruch 1, wobei das pflanzliche Rohmaterial ferner mindestens eines von *Artemisia argyi, Ophiopogon japonicus, Houttuynia cordata, Platycodon grandiflorus, Glycyrrhiza uralensis, Perilla frutescens* und Chrysantheme umfasst.

3. Pflanzliche Zusammensetzung zur Verwendung nach Anspruch 2, wobei das pflanzliche Rohmaterial, basierend auf dessen Gesamtgewicht, 18 bis 25 Gew.-% *Ohwia caudata* und mindestens eines von 18 bis 25 Gew.-% *Artemisia argyi,* 10 bis 17 Gew.-% *Ophiopogon japonicus,* 10 bis 17 Gew.-% *Houttuynia cordata,* 10 bis 17 Gew.-% *Platycodon grandiflorus,* 4 bis 11 Gew.-% *Glycyrrhiza uralensis,* 4 bis 11 Gew.-% *Perilla frutescens* und 0,4 bis 11 Gew.-% Chrysantheme umfasst, und/oder wobei das pflanzliche Material 5 g bis 7 g *Ohwia caudata* und mindestens eines von 5 g bis 7 g *Artemisia argyi,* 3 g bis 5 g *Ophiopogon japonicus,* 3 g bis 5 g *Houttuynia cordata,* 3 g bis 5 g *Platycodon grandiflorus,* 1 g bis 3 g *Glycyrrhiza uralensis,* 1 g bis 3 g *Perilla frutescens* und 0,1 g bis 3 g Chrysantheme umfasst.

4. Pflanzliche Zusammensetzung zur Verwendung nach Anspruch 1, wobei das Coronavirus das schwere akute respiratorische Syndrom Coronavirus (SARS-CoV), das Coronavirus des Nahost-Atemwegssyndroms (MERS-CoV), SARS-CoV-2, das Maus-Hepatitis-Virus (MHV) oder das Virus der epidemischen Schweinekrankheit (PEDV) ist.

5. Pflanzliche Zusammensetzung zur Verwendung nach Anspruch 4, wobei das Coronavirus eine Variante von SARS-CoV-2 ist.

6. Pflanzliche Zusammensetzung zur Verwendung nach Anspruch 1, wobei der Extrakt aus dem pflanzlichen Rohmaterial in der pflanzlichen Zusammensetzung dem Subjekt in einer wirksamen Menge von etwa 25 mg/kg/Tag bis etwa 2500 mg/kg/Tag verabreicht wird.

7. Pflanzliche Zusammensetzung zur Verwendung nach Anspruch 6, wobei der Extrakt aus dem pflanzlichen Rohmaterial in der pflanzlichen Zusammensetzung dem Subjekt in einer wirksamen Menge von etwa 50 mg/kg/Tag bis etwa 500 mg/kg/Tag verabreicht wird.

8. Pflanzliche Zusammensetzung zur Verwendung nach Anspruch 1, wobei die pflanzliche Zusammensetzung die Bindung eines Spike-Proteins eines Virus an einen Rezeptor des Angiotensin-konvertierenden Enzyms 2 (ACE2) stört.

9. Pflanzliche Zusammensetzung zur Verwendung nach Anspruch 1, wobei die pflanzliche Zusammensetzung die Expression von mindestens einem von 3CL-Protease, RNAabhängiger RNA-Polymerase (RdRp), transmembraner Serinprotease 2 (TRPMSS2) und FK506-Bindungsprotein 5 (FKBP5) unterdrückt.

## Revendications

1. Composition à base de plantes à utiliser dans la prévention ou le traitement d'une infection virale causée par un coronavirus, comprenant un extrait d'une matière première végétale et un support pharmaceutiquement acceptable, dans laquelle la matière première végétale comprend de l*'Ohwia caudata,* dans laquelle l*'Ohwia caudata* est une racine d*'Ohwia caudata,* ou une combinaison de la racine d'*Ohwia caudata* et d'une feuille d'*Ohwia caudata,* et dans laquelle l'extrait de la matière première végétale est un extrait d'eau.

2. Composition à base de plantes à utiliser selon la revendication 1, dans laquelle la matière première végétale comprend en outre au moins l'un parmi l*'Artemisia argyi, l'Ophiopogon japonicus,* l*'Houttuynia cordata, le Platycodon grandiflorus, la Glycyrrhiza uralensis,* la *Perilla frutescens* et le chrysanthème.

3. Composition à base de plantes à utiliser selon la revendication 2, dans laquelle la matière première végétale comprend, sur la base d'un poids total de celle-ci, 18 % à 25 % en poids d*'Ohwia caudata,* et au moins l'un parmi 18 % à 25 % en poids d'*Artemisia argyi,* 10 % à 17 % en poids d'*Ophiopogon japonicus,* 10 % à 17 % en poids d'*Houttuynia cordata,* 10 % à 17 % en poids de *Platycodon grandiflorus,* 4 % à 11 % en poids de *Glycyrrhiza uralensis,* 4 % à 11 % en poids de *Perilla frutescens,* et 0,4 % à 11 % en poids de chrysanthème, et/ou dans laquelle la matière première végétale comprend 5 g à 7 g d*'Ohwia caudata,* et au moins l'un parmi 5 g à 7 g d'*Artemisia argyi,* 3 g à 5 g d'*Ophiopogon japonicus,* 3 g à 5 g d'*Houttuynia cordata,* 3 g à 5 g de *Platycodon grandiflorus,* 1 g à 3 g de *Glycyrrhiza uralensis,* 1 g à 3 g de *Perilla frutescens,* et 0,1 g à 3 g de chrysanthème.

4. Composition à base de plantes à utiliser selon la revendication 1, dans laquelle le coronavirus est un coronavirus du syndrome respiratoire aigu sévère (SARS-CoV), un coronavirus du syndrome respiratoire du Moyen-Orient (MERS-CoV), un SARS-CoV-2, un virus de l'hépatite murine (MHV) ou un virus de la diarrhée épidémique porcine (PEDV).

5. Composition à base de plantes à utiliser selon la revendication 4, dans laquelle le coronavirus est un variant du SARS-CoV-2.

6. Composition à base de plantes à utiliser selon la revendication 1, dans laquelle l'extrait de la matière première végétale dans la composition à base de plantes est administré au sujet dans une quantité efficace d'environ 25 mg/kg/jour à environ 2 500 mg/kg/jour.

7. Composition à base de plantes à utiliser selon la revendication 6, dans laquelle l'extrait de la matière première végétale dans la composition à base de plantes est administré au sujet dans une quantité efficace d'environ 50 mg/kg/jour à environ 500 mg/kg/jour.

8. Composition à base de plantes à utiliser selon la revendication 1, dans laquelle la composition à base de plantes interfère avec la liaison d'une protéine de pointe d'un virus à un récepteur de l'enzyme de conversion de l'angiotensine 2 (ECA2).

9. Composition à base de plantes à utiliser selon la revendication 1, dans laquelle la composition à base de plantes supprime l'expression d'au moins l'une parmi la protéase 3CL, l'ARN polymérase ARN-dépendante (RdRp), la protéase transmembranaire à sérine 2 (TRPMSS2) et la protéine de liaison 5 FK506 (FKBP5).
